# EUROPÄISCHE PATENTANMELDUNG

(11) **EP 1 816 216 A2**
(43) Veröffentlichungstag der Anmeldung: **08.08.2007**
(21) Anmeldenummer: 07090045.1
(22) Anmeldetag: 24.11.2000
(51) Int. Cl.: C12Q 1/68

(54) **Oligomer-Array mit PNA-und/oder Oligomeren auf einer Oberfläche**

(30) Priorität: 25.11.1999 DE 19957827
(62) Teilanmeldung aus: 00993178.3
(71) Anmelder: Epigenomics AG, 10178 Berlin (DE)
(72) Erfinder: Berlin, Kurt, Dr., 14532 Stahnsdorf (DE)
(74) Vertreter: Schubert, Klemens

(57) **Zusammenfassung**

Beschrieben wird ein Oligomer-Array mit PNA- (Peptide Nucleic Acids)- und/oder DNA-Oligomeren auf einer Oberfläche, umfassend Oligomere aus jeweils zwischen 6 und 20 Monomeren oder Nukleobasen, wobei diese jeweils mindestens eine Sequenz der allgemeinen Formel DDCGDD oder der allgemeinen Formel DDTGDD oder der allgemeinen Formel HHCGHH oder der allgemeinen Formel HHCAHH umfassen,
wobei
H eine der Basen Adenin (A), Cytosin (C) oder Thymin (T) bedeutet und
D eine der Basen Adenin (A), Guanin (G) oder Thymin (T) darstellt,

und wobei der Ort der Oligomere auf der Oberfläche mit der Sequenz der Oligomere korreliert ist.

Die erfindungsgemäßen Oligomar-Arrays werden zur Detektion von Cytosin-Methylierungen in genomischer DNA verwendet.

## Beschreibung

Die Erfindung betrifft ein Oligomer-Array mit PNA- (Peptide Nucleic Acids)- und/oder DNA-Oligomeren auf einer Oberfläche.

Die nach den methodischen Entwicklungen der letzten Jahre in der Molekularbiologie gut studierten Beobachtungsebenen sind die Gene selbst, die Übersetzung dieser Gene in RNA und die daraus entstehenden Proteine. Wann im Laufe der Entwicklung eines Individuums welches Gen angeschaltet wird und wie Aktivieren und Inhibieren bestimmter Gene in bestimmten Zellen und Geweben gesteuert wird, ist mit Ausmaß und Charakter der Methylierung der Gene bzw. des Genoms korrelierbar. Insofern ist die Annahme naheliegend, daß pathogene Zustände sich in einem veränderten Methylierungsmuster einzelner Gene oder des Genoms äu-ßern.

5-Methylcytosin ist die häufigste kovalent modifizierte Base in der DNA eukaryotischer Zellen. Sie spielt beispielsweise eine Rolle in der Regulation der Transkription, genomischem Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüber hinaus geht bei einer PCR-Amplifikation die epigenetische Information, welche die 5-Methylcytosine tragen, vollständig verloren.

Die Modifikation der genomischen Base Cytosin zu 5'-Methylcytosin stellt den bis heute wichtigsten und bestuntersuchten epigenetischen Parameter dar. Trotzdem gibt es bis heute zwar Methoden, umfassende Genotypen von Zellen und Individuen zu ermitteln, aber noch keine vergleichbaren Ansätze auch in großem Maße epigenotypische Information zu generieren und auszuwerten.

Eine relativ neue und die mittlerweile am häufigsten angewandte Methode zur Untersuchung von DNA auf 5-Methylcytosin beruht auf der spezifischen Reaktion von Bisulphit mit Cytosin, das nach anschließender alkalischer Hydrolyse in Uracil umgewandelt wird, welches in seinem Basen-Paarungsverhalten dem Thymidin entspricht. 5-Methylcytosin wird dagegen unter diesen Bedingungen nicht modifiziert. Damit wird die ursprüngliche DNA so umgewandelt, daß Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese Techniken beruhen auf Basenpaarung, welche jetzt voll ausgenutzt werden kann. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulphit reagiert nur an einzelsträngiger DNA) verhindert und alle Fällungs- und Reinigungsschritte durch schnelle Dialyse ersetzt (Olek, A. et al., Nucl. Acids. Res. 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausende von möglichen Methylierungsereignissen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannte Möglichkeiten, 5-Methylcytsosine nachzuweisen, kann auch dem folgenden Übersichtsartikel entnommen werden: Rein, T., DePamphilis, M. L., Zorbas, H., Nucleic Acids Res. 26, 2255 (1998).

Es gibt grundsätzlich mehrere Möglichkeiten, Oligomer-Arrays auf den unterschiedlichsten Oberflächen herzustellen:
1. Alle Oligomere werden auf herkömmliche Weise einzeln und in relativ großer Menge im Reagenzglas bzw. in den speziellen Syntheseautomaten hergestellt und danach einzeln auf den Träger aufpipettiert. Dazu verwendet man üblicherweise automatische, hochpräzise Mikropipettierroboter. Der Vorteil dieses Verfahrens ist, daß es weitgehend auf bereits optimierten Standardmethoden und -Geräten beruht. Dadurch kann man qualitativ hochstehende DNA-Arrays mit sehr reinen Oligomeren herstellen, was einen äußerst positiven Einfluß auf die mit dem Array erzielbare Detektionsempfindlichkeit und -Zuverlässigkeit hat. Der große Nachteil des Verfahrens ist, daß es enorm aufwendig und deshalb teuer ist. Dies gilt in besonderem Masse für die Synthese der einzelnen Oligomere.
2. Die Oligomere werden durch Pipettieren in kleinsten Mengen direkt auf dem Substrat synthetisiert. Auf jedem Rasterpunkt wird die dort vorgesehene Oligomerkette Nukleobase für Nukleobase aufgebaut. Zur Pipettierung verwendet man ähnlich wie bei Verfahren 1) einen spezialisierten Mikropipettierroboter oder z. B. eine Vorrichtung, die Kanäle zur Zuführung der einzelnen Synthesebausteine zu den jeweiligen Punkten des Arrays enthält (EP-A 0915897). Das chemische Syntheseverfahren ist grundsätzlich das gleiche wie bei der herkömmlichen Oligomer-Synthese im Syntheseautomaten. Der Unterschied ist, daß alle Oligomere unabhängig von deren Anzahl gleichzeitig, von einer einzigen automatischen Anlage direkt am vorgesehenen Bestimmungsort hergestellt werden. Die bei Methode 1) separaten Arbeitsschritte Oligomer-Synthese und Mikropipettierung sind nun zu einem einzigen Arbeitsschritt zusammengefaßt. Der Aufwand an Geräten und an manueller Arbeit wird gegenüber Methode 1) erheblich reduziert.
3. Die Oligomere werden wie bei Methode 2) direkt auf dem Substrat synthetisiert, die gezielte Anbindung der richtigen Nukleobasen an den richtigen Rasterpunkten geschieht jedoch durch eine vollkommen parallele, aus der Halbleiterfertigung stammende photolithographische Technik anstelle von sequentiellen, zielgenauen Pipettierschritten. Das Verfahren basiert darauf, daß man mit Licht einer bestimmten Wellenlänge gezielt die 5'-OH Schutzgruppen von Oligonukleotiden entfernen kann. Durch geeignete örtliche Bestrahlungsmuster kann man somit Oligonukleotid-Enden an genau jenen Rasterpunkten reaktionsfähig machen, an die man im nächsten Schritt einen neuen Nukleotidbaustein anbinden will. Bei vollständiger Benetzung der Arrayoberfläche mit einer Nukleotidbaustein-Lösung wird somit nur an den vorher belichteten Stellen eine Nukleobase angebunden, alle unbelichteten Stellen bleiben unverändert. Die örtlichen Belichtungsmuster werden erzeugt, indem man eine mikrophotographische schwarzweiß Maske zwischen dem Substrat und der Lichtquelle positioniert, die alle Rasterstellen abdeckt, die nicht reaktionsfähig gemacht werden sollen. Die Verlängerung der Oligomer-Ketten auf allen Rasterpunkten um eine Nukleobase geschieht demnach wie folgt: Mit Hilfe einer ersten Maske werden genau jene Rasterpunkt belichtet, welche um die erste der 4 möglichen Sorten von Nukleobasen (z.B. C) erweitert werden müssen. Danach wird der Array mit einer Lösung des entsprechenden Nukleotidbausteins benetzt, worauf nur die belichteten Punkte um diese Base verlängert werden. Da die neu angebundenen Nukleotidbausteine noch alle über eine Schutzgruppe verfügen, werden sie in den folgenden Schritten nicht weiter reagieren, bis ihre Schutzgruppen durch eine weitere Belichtung abgespaltet werden. Nach diesem Reaktionsschritt wird das Array gewaschen. Nun werden mit Hilfe einer zweiten Maske genau jene Rasterstellen belichtet, welche um die zweite der 4 möglichen Nukleobasen (z.B. T) erweitert werden müssen. Darauf wird das Array wiederum mit einer Lösung des entsprechenden Nukleotidbausteins benetzt und die belichteten Stellen dadurch um diese Base verlängert. Genauso verfährt man für die verbleibenden zwei Nukleobasen (G und A). Für die Verlängerung aller Oligomere um eine Nukleobase benötigt man demzufolge vier Belichtungsschritte und somit 4 Photomasken.

Diese Methode ist wegen der hohen Parallelität in der Verarbeitung sehr schnell und effizient, zudem ist sie wegen der hohen Präzision, die mit Photolithographie erreicht werden kann, gut dazu geeignet, sehr hohe Rasterdichten zu erzielen.

Eine Übersicht über den Stand der Technik in der Oligomer Array Herstellung läßt sich auch einer im Januar 1999 erschienen Sonderausgabe von Nature Genetics (Nature Genetics Supplement, Volume 21, January 1999) und der dort zitierten Literatur entnehmen.

Patente, die sich allgemein auf die Verwendung von Oligomer Arrays und photolithographisches Maskendesign beziehen, sind z. B. US-A 5,837,832, US-A 5,856,174, WO-A 98/27430 und US-A 5,856,101. Zudem existieren einige Stoff- und Verfahrenspatente, welche die Verwendung photolabiler Schutzgruppen an Nukleosiden einschränken, so z. B. WO-A98/39348 und US-A 5,763,599.

Um DNA zu immobilisieren, existieren verschiedene Verfahren. Das bekannteste Verfahren ist die Festbindung einer DNA, welche mit Biotin funktionalisiert ist, an eine Streptavidin-beschichtete Oberfläche. Die Bindungsstärke dieses Systems entspricht einer kovalenten chemischen Bindung ohne eine zu sein. Um eine Ziel-DNA kovalent an eine chemisch vorbereitete Oberfläche binden zu können, bedarf es einer entsprechenden Funktionalität der Ziel-DNA. DNA selbst besitzt keine Funktionalisierung, die geeignet ist. Es gibt verschiedene Varianten in eine Ziel-DNA eine geeignete Funktionalisierung einzuführen: Zwei leicht zu handhabende Funktionalisierungen sind primäre, aliphatische Amine und Thiole. Solche Amine werden quantitativ mit N-Hydroxy-succinimidestern umgesetzt und Thiole reagieren unter geeigneten Bedingungen quantitativ mit Alkyliodiden. Eine Schwierigkeit besteht im Einführen einer solchen Funktionalisierung in eine DNA. Die einfachste Variante ist die Einführung durch einen Primer einer PCR. Gezeigte Varianten benützen 5'-modifizierte Primer (NH2 und SH) und einen bifunktionalen Linker.

Ein wesentlicher Bestandteil der Immobilisierung auf einer Oberfläche ist ihre Beschaffenheit. Bis jetzt beschriebene Systeme sind hauptsächlich aus Silizium oder Metall (magnetic beads). Eine weitere Methode zur Bindung einer Ziel-DNA basiert darauf, eine kurze Erkennungssequenz (z.B. 20 Basen) in der Ziel-DNA zur Hybridisierung an ein oberflächenimmobilisiertes Oligonukleotid zu verwenden.

Als Sonden, die in einem Oligomer-Array auf einer Oberfläche fixiert werden, kommen Oligonukleotide in Frage, jedoch bietet sich auch jede Modifikation von Nukleinsäuren an, z.B. Peptide Nucleic Acids (PNA), (Nielsen, P.E., Buchardt, O., Egholm, M. and Berg, R.H. 1993. Peptide nucleic acids. US Patent. 5,539,082; Buchardt, O., Egholm, M., Berg, R.H. and Nielsen, P.E. 1993. Peptide nucleic acids and their potential applications in biotechnology. Trends in Biotechnology, 11: 384-386), Phosphorothioatoligonukleotide oder Methylphosphonatoligonukleotide. Die Spezifität einer Sonde ist sehr wesentlich. Peptide Nucleic Acids haben ein ungeladenes Rückgrat, welches gleichzeitig chemisch sehr stark von der gängigen Zucker-Phosphat Struktur des Rückgrats in Nukleinsäuren abweicht. Das Rückgrat einer PNA hat eine Amidsequenz anstelle des Zucker-Phosphat Rückgrats gewöhnlicher DNA. PNA hybridisiert sehr gut mit DNA komplementärer Sequenz. Die Schmelztemperatur eines PNA/DNA-Hybrids ist höher als die des entsprechenden DNA/DNA-Hybrids und die Abhängigkeit der Hybridisierung von Puffersalzen ist relativ gering.

Matrix-assistierte Laser Desorptions/Ionisations Massenspektrometrie (MALDI) ist eine neue, sehr leistungsfähige Entwicklung für die Analyse von Biomolekülen (Karas, M. and Hillenkamp, F. 1988. Laser desorption ionization of proteins with molecular masses exceeding 10.000 daltons. Anal. Chem. 60: 2299-2301). Ein Analytmolekül wird in eine im UV absorbierende Matrix eingebettet. Durch einen kurzen Laserpuls wird die Matrix ins Vakuum verdampft und das Analyt so unfragmentiert in die Gasphase befördert. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere und die Flugzeit wird in die Masse der Ionen umgerechnet.

Aufgabe der vorliegenden Erfindung ist es, Oligomer-Arrays bereitzustellen, welche sich für die Detektion von Cytosin Methylierungen besonders eignen.

Die Aufgabe wird erfindungsgemäß dadurch gelöst, daß ein Oligomer-Array mit PNA- (Peptide Nucleic Acids)- und/oder DNA-Oligomeren auf einer Oberfläche geschaffen wird, umfassend Oligomere aus jeweils zwischen 6 und 20 Monomeren oder Nukleobasen, wobei diese jeweils mindestens eine Sequenz der allgemeinen Formel DDCGDD oder der allgemeinen Formel DDTGDD oder der allgemeinen Formel HHCGHH oder der allgemeinen Formel HHCAHH umfassen,
wobei
H eine der Basen Adenin (A), Cytosin (C) oder Thymin (T) bedeutet und
D eine der Basen Adenin (A), Guanin (G) oder Thymin (T) darstellt,
und wobei der Ort der Oligomere auf der Oberfläche mit der Sequenz der Oligomere korreliert ist.

Erfindungsgemäß bevorzugt ist es, dass mindestens 10 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

Erfindungsgemäß bevorzugt ist es auch, dass mindestens 25 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

Erfindungsgemäß ist weiterhin auch bevorzugt, dass mindestens 50 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

Erfindungsgemäß bevorzugt ist gleichfalls, dass mindestens 75 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

Erfindungsgemäß bevorzugt ist es, daß die Oberfläche eben ist und die Oligomere in einem rechtwinkligen oder hexagonalen Raster, das die Zuordnung zu Koordinaten erlaubt, darauf angeordnet sind. Es können aber auch andere zweckmäßige geometrische Anordnungen gewählt werden, welche die Möglichkeiten der Automatisierung verbessern helfen wie beispielsweise circulare Anordnungen.

Bevorzugt ist ein Oligomer-Array umfassend Sequenzen der allgemeinen Formel DDCGDD und der allgemeinen Formel DDTGDD und der allgemeinen Formel HHCGHH und der allgemeinen Formel HHCAHH.

Besonders bevorzugt ist ein Oligomer-Array umfassend Sequenzen der allgemeinen Formel DDCGDD und der allgemeinen Formel DDTGDD oder Sequenzen der allgemeinen Formel HHCGHH und der allgemeinen Formel HHCAHH.

Bevorzugt ist es dabei, daß dieses mindestens 100 verschiedene Oligomere umfaßt.

Erfindungsgemäß bevorzugt ist ein erfindungsgemäßes Oligomer-Array welches dadurch gekennzeichnet ist, dass passend zu jedem Oligomer, welches eine CG-Sequenz umfasst, ein analoges Oligomer immobilisiert ist, welches sich vom besagten Oligomer nur dadurch unterscheidet, dass es anstelle der CG-Sequenz eine TG- oder aber eine CA-Sequenz enthält.

Es ist ferner bevorzugt, daß die Oberfläche aus Glas ist.

Bevorzugt ist auch, daß die Oberfläche aus Metall oder einem anderen leitfähigen Material ist. Ganz besonders bevorzugt ist dabei, daß die Oberfläche das Target eines MALDI-Massenspektrometers ist.

Die vorliegende Erfindung beschreibt somit Oligomer-Arrays, die zur Detektion des Methylierungszustandes genomischer DNA Proben verwendbar sind.

Ein weiterer Gegenstand der vorliegenden Erfindung ist daher die Verwendung eines erfindungsgemäßen Oligomer-Arrays zur Hybridisierung von DNA-Fragmenten nach vorheriger Amplifikation.

Besonders bevorzugt ist es dabei, daß man die DNA vor der Amplifikation mit einer Bisulfitlösung (oder Hydrogensulfit-, Disulfitlösung) behandelt.

Ein weiterer Gegenstand der vorliegenden Erfindung ist auch die Verwendung eines erfindungsgemäßen Oligomer-Arrays zur Detektion von Cytosin-Methylierungen in genomischer DNA.

Gegenstand der vorliegenden Erfindung ist also eine Anordnung, vorzugsweise in Form eines Oligomer-Arrays, von PNA (Peptide Nucleic Acids)- oder DNA-Oligomeren auf einer Oberfläche, umfassend Oligomere aus jeweils zwischen 6 und 20 Monomeren (oder Nukleobasen), die wiederum Sequenzen der allgemeinen Formel DDCGDD und/oder der allgemeinen Formel DDTGDD und/oder der allgemeinen Formel HHCGHH und/oder der allgemeinen Formel HHCAHH umfassen,
wobei der Ort der Oligomere auf der Oberfläche jeweils einen Rückschluß auf ihre Sequenz(en) erlaubt. Oligomer-Arrays dieses Typs sind besonders zur Detektion von Cytosin Methylierungen in genomischer DNA geeignet. Die oben aufgeführten Sequenzen hybridisieren je nach Methylierungsstatus der DNA nach deren chemischer Vorbehandlung mit Bisulfit unterschiedlich stark.

Um die von diesen Hybridisierungen ausgehenden Signale besser den eingesetzten Oligomersequenzen zuordnen zu können, ist es besonders bevorzugt, daß die Oberfläche eben ist und die Oligomere in einem rechtwinkligen oder hexagonalen Raster, das die Zuordnung zu Koordinaten erlaubt, darauf angeordnet sind.

Besonders bevorzugt ist eine Anordnung von PNA (Peptide Nucleic Acids)- oder DNA-Oligomeren auf einer Oberfläche, umfassend Oligomere aus jeweils zwischen 6 und 20 Monomeren (oder Nukleobasen), umfassend Sequenzen der allgemeinen Formel DDCGDD und der allgemeinen Formel DDTGDD und der allgemeinen Formel HHCGHH und der allgemeinen Formel HHCAHH, wobei der Ort der Oligomere auf der Oberfläche jeweils einen Rückschluß auf ihre Sequenz(en) erlaubt.

Besonders bevorzugt ist eine Anordnung von PNA (Peptide Nucleic Acids)- oder DNA-Oligomeren auf einer Oberfläche, umfassend Oligomere aus jeweils zwischen 6 und 20 Monomeren (oder Nukleobasen) der allgemeinen Formel DDCGDD und der allgemeinen Formel DDTGDD, wobei der Ort der Oligomere auf der Oberfläche jeweils einen Rückschluß auf ihre Sequenz(en) erlaubt.

Besonders bevorzugt ist eine Anordnung von PNA (Peptide Nucleic Acids)- oder DNA-Oligomeren auf einer Oberfläche, umfassend Oligomere aus jeweils zwischen 6 und 20 Monomeren (oder Nukleobasen) der allgemeinen Formel HHCGHH und der allgemeinen Formel HHCAHH, wobei der Ort der Oligomere auf der Oberfläche jeweils einen Rückschluß auf ihre Sequenz(en) erlaubt.

Besonders bevorzugt ist es zudem, daß die Anordnung mindestens 100 verschiedene Oligomere umfaßt, die jeweils mindestens eine der Sequenzen DDCGDD, DDTGDD, HHCGHH oder HHCAHH umfassen.

In einer besonders bevorzugten Ausführung besteht die Oberfläche der Anordnung aus Glas. In einer weiteren bevorzugten Ausführung besteht die Oberfläche der Anordnung aus Metall oder einem anderen leitfähigen Material. In einer ebenfalls besonders bevorzugten Ausführung ist die Anordnung dadurch gekennzeichnet, daß die Oberfläche das Target eines MALDI-Massenspektrometers ist.

Gegenstand der vorliegenden Erfindung ist zudem die Verwendung einer Anordnung von PNA (Peptide Nucleic Acids)- oder DNA-Oligomeren auf einer Oberfläche, umfassend Oligomere aus jeweils zwischen 6 und 20 Monomeren (oder Nukleobasen), die wiederum Sequenzen der allgemeinen Formel DDCGDD und/oder der allgemeinen Formel DDTGDD und/oder der allgemeinen Formel HHCGHH und/oder der allgemeinen Formel HHCAHH umfassen, wobei der Ort der Oligomere auf der Oberfläche jeweils einen Rückschluß auf ihre Sequenz(en) erlaubt, zur Hybridisierung von DNA-Fragmenten, die zuvor amplifiziert wurden.

Besonders bevorzugt ist dabei der Einsatz von DNA-Fragmenten, die mittels der Polymerase-Kettenreaktion hergestellt wurden.

Besonders bevorzugt ist zudem die Verwendung einer Anordnung von PNA (Peptide Nucleic Acids)- oder DNA-Oligomeren auf einer Oberfläche, umfassend Oligomere aus jeweils zwischen 6 und 20 Monomeren (oder Nukleobasen), die wiederum Sequenzen der allgemeinen Formel DDCGDD und/oder der allgemeinen Formel DDTGDD und/oder der allgemeinen Formel HHCGHH und/oder der allgemeinen Formel HHCAHH umfassen, wobei der Ort der Oligomere auf der Oberfläche jeweils einen Rückschluß auf ihre Sequenz(en) erlaubt, zur Hybridisierung von DNA, die zuvor mit einer Bisulfitlösung (oder Hydrogensulfit, Disulfit) behandelt wurde. Besonders bevorzugt wurde die DNA amplifiziert.

Besonders bevorzugt ist zudem die Verwendung einer Anordnung von PNA (Peptide Nucleic Acids)- oder DNA-Oligomeren auf einer Oberfläche, umfassend Oligomere aus jeweils zwischen 6 und 20 Monomeren (oder Nukleobasen), die wiederum Sequenzen der allgemeinen Formel DDCGDD und/oder der allgemeinen Formel DDTGDD und/oder der allgemeinen Formel HHCGHH und/oder der allgemeinen Formel HHCAHH umfassen, wobei der Ort der Oligomere auf der Oberfläche jeweils einen Rückschluß auf ihre Sequenz(en) erlaubt, für die Detektion von Cytosin-Methylierungen in genomischer DNA.

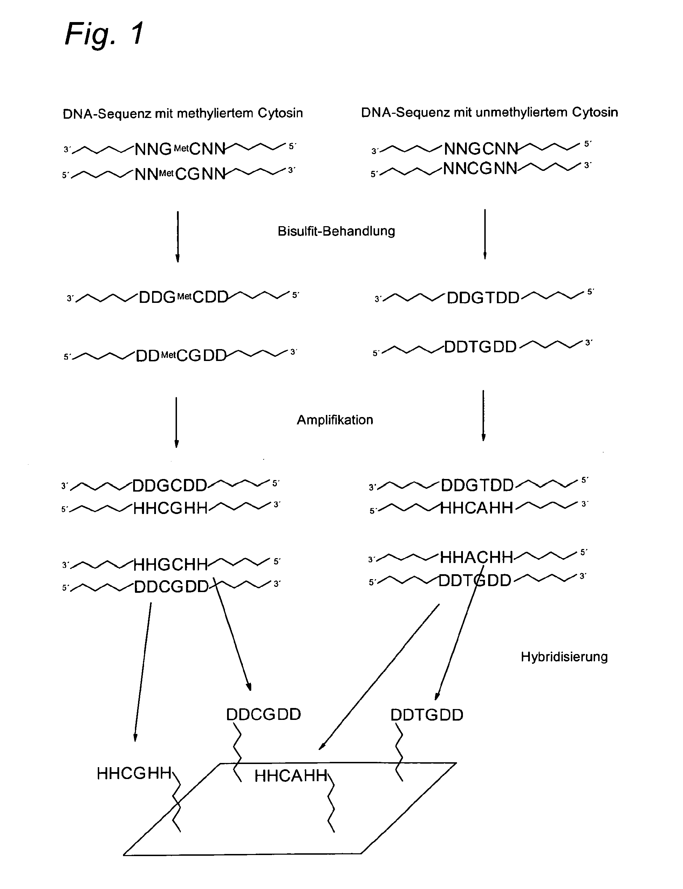

In der Figur 1 ist die erfindungsgemäße Verwendung der erfindungsgemäßen Oligomer-Arrays zur Detektion von Cytosin-Methylierungsmustern in genomischer DNA beispeielhaft erläutert.

In der Figur 1 haben die Buchstaben H, D und N die folgende Bedeutung:
- H: stellt eine der Basen Adenin (A), Cytosin (C) oder Thymin (T),
- D: eine der Basen Adenin (A), Guanin (G) oder Thymin (T) und
- N: eine der Basen Adenin (A), Guanin (G), Cytosin (C) oder Thymin (T) dar.

DNA-Sequenzen, welche sich lediglich in der Methylierung von Cytosin unterscheiden, ergeben nach der Behandlung mit Bisulfit eine veränderte Sequenz der Nukleobasen. Das methylierte Cytosin wird durch die Bisulfitbehandlung nicht verändert, während das nicht methylierte Cytosin zu Thymin umgewandelt wird. Dies führt nach der Amplifikation zu unterschiedlichen Sequenzen, welche dann an verschiedenen Stellen des Oligomer-Arrays binden, an denen komplementäre Sequenzen vorhanden sind. Somit ist, da die Sequenzen auf dem Oligomer-Array bekannt sind, ein Rückschluß auf eine in der ursprünglichen DNA vorhandenen Methlylierung des Cytosins möglich.

## Patentansprüche

1. Oligomer-Array mit PNA- (Peptide Nucleic Acids)- und/oder DNA-Oligomeren auf einer Oberfläche, umfassend Oligomere aus jeweils zwischen 6 und 20 Monomeren oder Nukleobasen, wobei diese jeweils mindestens eine Sequenz der allgemeinen Formel DDCGDD oder der allgemeinen Formel DDTGDD oder der allgemeinen Formel HHCGHH oder der allgemeinen Formel HHCAHH umfassen,
wobei
H eine der Basen Adenin (A), Cytosin (C) oder Thymin (T) bedeutet und
D eine der Basen Adenin (A), Guanin (G) oder Thymin (T) darstellt,
und wobei der Ort der Oligomere auf der Oberfläche mit der Sequenz der Oligomere korreliert ist.

2. Oligomer-Array nach Anspruch 1, **dadurch gekennzeichnet, dass** mindestens 10 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

3. Oligomer-Array nach Anspruch 1, **dadurch gekennzeichnet, dass** mindestens 25 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

4. Oligomer-Array nach Anspruch 1, **dadurch gekennzeichnet, dass** mindestens 50 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

5. Oligomer-Array nach Anspruch 1, **dadurch gekennzeichnet, dass** mindestens 75 % der Oligonucleotide eine Sequenz der allgemeinen Formel DDCGDD oder der allgemeinen Formel DDTGDD oder der allgemeinen Formel HHCGHH oder der allgemeinen Formel HHCAHH umfassen.

6. Oligomer-Array nach einem der Ansprüche 1 bis 5, **dadurch gekennzeichnet, daß** die Oberfläche eben ist und die Oligomere in einem rechtwinkligen oder hexagonalen Raster, das die Zuordnung zu Koordinaten erlaubt, darauf angeordnet sind.

7. Oligomer-Array nach einem der voranstehenden Ansprüche, umfassend Sequenzen der allgemeinen Formel DDCGDD und der allgemeinen Formel DDTGDD und der allgemeinen Formel HHCGHH und der allgemeinen Formel HHCAHH.

8. Oligomer-Array nach einem der Ansprüch 1 bis 6, umfassend Sequenzen der allgemeinen Formel DDCGDD und der allgemeinen Formel DDTGDD oder Sequenzen der allgemeinen Formel HHCGHH und der allgemeinen Formel HHCAHH.

9. Oligomer-Array nach einem der voranstehenden Ansprüche, **dadurch gekennzeichnet, daß** dieses mindestens 100 verschiedene Oligomere umfaßt.

10. Oligomer-Array nach einem der voranstehenden Ansprüche, **dadurch gekennzeichnet, dass** passend zu jedem Oligomer, welches eine CG-Sequenz umfasst, ein analoges Oligomer immobilisiert ist, welches sich vom besagten Oligomer nur **dadurch** unterscheidet, dass es anstelle der CG-Sequenz eine TG- oder aber eine CA-Sequenz enthält.

11. Oligomer-Array nach einem der voranstehenden Ansprüche, **dadurch gekennzeichnet, daß** die Oberfläche aus Glas ist.

12. Oligomer-Array nach einem der Ansprüche 1 bis 10, **dadurch gekennzeichnet, daß** die Oberfläche aus Metall oder einem anderen leitfähigen Material ist.

13. Oligomer-Array nach Anspruch 12, **dadurch gekennzeichnet, daß** die Oberfläche das Target eines MALDI-Massenspektrometers ist.

14. Verwendung eines Oligomer-Arrays nach Anspruch 1 zur Hybridisierung von DNA-Fragmenten nach vorheriger Amplifikation.

15. Verwendung gemäß Anspruch 14, **dadurch gekennzeichnet, daß** man die DNA vor der Amplifikation mit einer Bisulfitlösung (oder Hydrogensulfit-, Disulfitlösung) behandelt.

16. Verwendung eines Oligomer-Arrays nach Anspruch 1 zur Detektion von Cytosin-Methylierungen in genomischer DNA.
